# EUROPEAN PATENT APPLICATION

(11) **EP 4 674 948 A1**
(43) Date of publication of application: **07.01.2026**
(21) Application number: 24852929.9
(22) Date of filing: 08.07.2024
(51) Int. Cl.: C12N 1/20, A61K 35/741, A61K 35/745, A61P 3/04, A61P 3/06, A61P 3/10, A23L 33/135, C12R 1/01

(54) **AKKERMANSIA MUCINIPHILA AKK11 HAVING FUNCTIONS OF REGULATING BLOOD GLUCOSE AND BLOOD FAT, AND USE THEREOF**

(30) Priority: 20.05.2024 CN 202410620733
(71) Applicant: Wecare Probiotics Co., Ltd., Suzhou, Jiangsu 215200 (CN)
(72) Inventor: FANG, Shuguang, Suzhou, Jiangsu 215200 (CN); TANG, Haifeng, Suzhou, Jiangsu 215200 (CN); DONG, Yao, Suzhou, Jiangsu 215200 (CN); ZUO, Huiyu, Suzhou, Jiangsu 215200 (CN); WU, Zhiyi, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Buzzi, Notaro & Antonielli d'Oulx S.p.A.
(86) International application number: PCT/CN2024/104282
(87) International publication number: WO 2025/241274

(57) **Abstract**

Provided are an *Akkermansia muciniphila* Akk11 with the effect of regulating blood glucose and blood lipid, and a use thereof. The *Akkermansia muciniphila* Akk11 with the effect of regulating blood glucose and blood lipid has a taxonomic name of *Akkermansia muciniphila* Akk11, with a deposit number CCTCC NO: M 2024119. The strain uses mucin as the only source of carbon and nitrogen elements to control the basic regulatory system of glucose and energy metabolism, which can protect the integrity of intestinal epithelial cells and mucus layer and play a metabolic protective role, regulate blood glucose and blood lipid levels, significantly improve symptoms of metabolic disorders, and provide coping strategies and references for the prevention and treatment of obesity.

## Description

### TECHNICAL FIELD

The present application belongs to the technical field of microbial cultivation, and relates to an *Akkermansia muciniphila* AKK11 with the effect of regulating blood glucose and blood lipid, and a use thereof.

### BACKGROUND

*Akkermansia muciniphila* (Akk) is a strict anaerobic bacteria, which is often colonized in the intestines of humans and other animals. The *Akkermansia muciniphila* is negatively associated with metabolic disorders such as metabolic endotoxemia, obesity-related insulin resistance and glucose tolerance. *Akkermansia muciniphila* uses mucin as the only source of carbon and nitrogen elements, and controls a basic regulatory system of glucose and energy metabolism, which can not only protect the integrity of intestinal epithelial cells and mucous layers to play a metabolic protective role, but can also play an anti-inflammatory role through regulatory T cells, endocannabinoid system, and non-classical Toll-like receptors during the inflammatory response.

Obesity is a disease caused by metabolic problems. Traditionally, obesity is defined as the accumulation of visceral and subcutaneous fat and weight gain, which may be detrimental to health. In different stages of growth, the *Akkermansia muciniphila* is colonized in human intestine. For the infancy period, *Akkermansia muciniphila* can be used as a marker of the growth status and diversity of the intestinal microbiota; and for the process of early stage of life to adulthood, the quantity of *Akkermansia muciniphila* increased significantly, and the detected abundance of *Akkermansia muciniphila* will be significantly reduced in the case of obesity and diabetes.

Probiotics are defined as "living microorganisms" that have a health impact on the host when ingested in sufficient quantities. In the case where the obesity is caused by excessive energy intake from food and metabolic-induced energy balance problem, the species and quantity of microorganism in the host intestine will both change, making probiotics possible as a potential target. Probiotics can improve lipid metabolism disorders, inflammation, oxidative stress and intestinal microecological disorders caused by high-fat diet, and prevent, improve or treat obesity by improving the abundance of intestinal microbiota. Therefore, how to provide an efficient microbial preparation containing probiotics to improve symptoms such as metabolic obesity has become an urgent technical problem.

### SUMMARY

The present application provides an *Akkermansia muciniphila* Akk11 with the effect of regulating blood glucose and blood lipid, and a use thereof, in particular to an *Akkermansia muciniphila* Akk11 with the effect of regulating blood glucose and blood lipid, and a use thereof in the preparation of a product with the effect of preventing, improving, or treating obesity, hyperlipidemia, and hyperglycemia.

In a first aspect, the present application provides an *Akkermansia muciniphila* Akk11 with the effect of regulating blood glucose and blood lipid. The *Akkermansia muciniphila* Akk11 with the effect of regulating blood glucose and blood lipid is taxonomy named as *Akkermansia muciniphila* Akk11 and deposited on Jan. 15, 2024, with a deposit number CCTCC NO: M 2024119.

In the present application, a new strain of *Akkermansia muciniphila* with the effect of regulating blood glucose and blood lipid is isolated and deposited from a healthy infant fecal sample, which is named as *Akkermansia muciniphila* Akk11. The strain can regulate the level of blood glucose and blood lipid and significantly improve symptoms such as metabolic obesity, specifically manifested in: (1) significantly improving the ability to decompose and utilize glucose, and improving the disorder of glucose metabolic; (2) significantly reducing the indicators such as total cholesterol, triglycerides, and low-density lipoprotein cholesterol; and (3) effectively regulating the gastrointestinal inflammation caused by metabolic obesity and other factors, and having a significant improvement effect on inflammatory factors. Therefore, the *Akkermansia muciniphila* Akk11 strain can be used in the preparation of a product with the effect of preventing, improving, or treating obesity, hyperlipidemia, and hyperglycemia.

In addition, *Akkermansia muciniphila* is a probiotic, therefore, the *Akkermansia muciniphila* Akk11 screen out by the present application has a high safety when used in the preparation of related functional products; and the strain will not develop drug-resistance, which can improve the occurrence of endocrine disruption, nausea, and other adverse effects associated with antibiotic administration in routinely obese patients, and can be used in a product for long-term preventing, improving, or treating metabolic obesity.

In a second aspect, the present application provides a culture of the *Akkermansia muciniphila* Akk11 with the effect of regulating blood glucose and blood lipid as described in the first aspect. The culture is prepared by the following method: inoculating the *Akkermansia muciniphila* Akk11 strain in a medium and culturing at 35-38°C for 18-22 h.

The "35-38°C", which may be, for example, 35°C, 35.5°C, 36°C, 36.5°C, 37°C, 37.5°C or 38°C. Other specific point values within the above numeric range are also applicable, which are not recited here for brevity.

The "18-22 h", which may be, for example, 18 h, 18.5 h, 19 h, 19.5 h, 20 h, 20.5 h, 21 h, 21.5 h or 22 h. Other specific point values within the above numeric range are also applicable, which are not recited here for brevity.

In a third aspect, the present application provides a probiotic preparation with the effect of regulating blood glucose and blood lipid. The probiotic preparation with the effect of regulating blood glucose and blood lipid includes the *Akkermansia muciniphila* Akk11 strain as described in the first aspect.

Preferably, a viable bacteria count of the *Akkermansia muciniphila* Akk11 strain in the probiotic preparation is not less than 1×10¹⁰ CFU/mL or 1×10¹⁰ CFU/g, for example, 1×10¹⁰ CFU/mL (CFU/g), 2×10¹⁰ CFU/mL (CFU/g), 5×10¹⁰ CFU/mL (CFU/g), 8×10¹⁰ CFU/mL (CFU/g), 1×10¹¹ CFU/mL (CFU/g), 5×10¹¹ CFU/mL (CFU/g) or 1×10¹² CFU/mL (CFU/g). Other specific point values within the above numeric range are also applicable, which are not recited here for brevity.

Preferably, the probiotic preparation with the effect of regulating blood glucose and blood lipid further includes *Bifidobacterium longum* BL21. The *Bifidobacterium longum* BL21 is taxonomy named as *Bifidobacterium longum* and deposited on Jan. 27, 2015, with a deposit number CGMCC No.10452.

The present application also creatively found that the *Akkermansia muciniphila* Akk11 strain is able to be compounded with the *Bifidobacterium longum* BL21 strain for the improvement of obesity, which has a significantly superior result than a single microbial preparation or other compounding methods, indicating a synergistic effect of the Akk11 strain and the BL21 strain in the aspects such as enhancing the ability of glucose decomposition and metabolism, improving the conditions of glucose metabolic disorders, reducing the indicators such as total cholesterol, triglycerides, and low-density lipoprotein cholesterol, and effectively regulating the gastrointestinal inflammation caused by metabolic obesity and other factors.

Preferably, a viable bacteria count ratio of the *Akkermansia muciniphila* Akk11 strain to the *Bifidobacterium longum* BL21 strain is (1:10)-(10:1); for which, "1-10" may be, for example,1, 2, 3, 4, 5, 6, 7, 8, 9, or 10. Other specific point values within the above numeric range are also applicable, which are not recited here for brevity.

Preferably, a dosage form of the probiotic preparation includes a lyophilized powder agent, a capsule agent, a tablet agent or a granule agent.

Preferably, the probiotic preparation further includes a protectant and/or a functional additive.

Preferably, the protectant includes any one or a combination of at least two of skimmed milk, gelatin, dextrine, Arabic gum, dextran, sodium algae, polyvinyl pyrrolidone, sucrose, lactose, trehalose, sorbitol or xylitol.

Preferably, the functional additive includes any one or a combination of at least two of maltodextrin, oligofructose, oligogalactose, oligoxylose, soybean oligosaccharide, inulin, *Spirulina, Arthrospira,* Coriolus versicolor polysaccharide, stachyose, polyglucose, α-lactalbumin, or lactoferrin.

In a forth aspect, the present application provides a use of the *Akkermansia muciniphila* Akk11 with the effect of regulating blood glucose and blood lipid as described in the first aspect, or the culture described in the second aspect, or the probiotic preparation described in the third aspect in the preparation of a product with the effect of preventing, improving, or treating obesity, hyperlipidemia, and hyperglycemia.

Preferably, the product further includes an auxiliary material.

Preferably, the auxiliary material includes any one or a combination of at least two of a diluent, an excipient, a binder, a wetting agent, a disintegrant, an emulsifier, a cosolvent, a solubilizer, an osmotic pressure regulator, a coating material, a colorant, a pH regulator, or a buffer.

The Akk11 strain of the present application is taxonomy named as *Akkermansia muciniphila* Akk11 and deposited in China Center for Type Culture Collection on Jan. 15, 2024, with a deposit number CCTCC NO: M 2024119, and the deposited address is Wuhan University, Wuhan, China.

The BL21 strain of the present application is taxonomy named as *Bifidobacterium longum* and deposited in China General Microbiological Culture Collection Center on Jan. 27, 2015, with a deposit number CGMCC No.10452, and the deposited address is No. 3, Courtyard 1, West Beichen Road, Chaoyang District, Beijing.

Compared to the prior art, the present application has the beneficial effects as follows.

In the present application, a new strain of *Akkermansia muciniphila* with the effect of regulating blood glucose and blood lipid is isolated and deposited, which is named as *Akkermansia muciniphila* Akk11. The strain can regulate the level of blood glucose and blood lipid and significantly improve symptoms such as metabolic obesity, specifically manifested in: (1) significantly improving the ability to decompose and utilize glucose, and improving the disorder of glucose metabolic; (2) significantly reducing the indicators such as total cholesterol, triglycerides, and low-density lipoprotein cholesterol; and (3) effectively regulating the gastrointestinal inflammation caused by metabolic obesity and other factors, and having a significant improvement effect on inflammatory factors. Therefore, the *Akkermansia muciniphila* Akk11 strain can be used in the preparation of a product with the effect of preventing, improving, or treating obesity, hyperlipidemia, and hyperglycemia.

### BRIEF DESCRIPTION OF DRAWINGS

FIG. 1 is a graph showing the changes of oral glucose tolerance with time in mice of each group.
FIG. 2 is a graph showing the results of total cholesterol in serum of mice in each group.
FIG. 3 is a graph showing the results of triglycerides in serum of mice in each group.
FIG. 4 is a graph showing the results of high-density lipoprotein cholesterol in serum of mice in each group.
FIG. 5 is a graph showing the results of low-density lipoprotein cholesterol in serum of mice in each group.
FIG. 6 is a graph showing the results of inflammatory factor IL-2 in pancreatic cells of mice in each group.
FIG. 7 is a graph showing the results of inflammatory factor IL-6 in pancreatic cells of mice in each group.
FIG. 8 is a graph showing the results of inflammatory factor IL-10 in pancreatic cells of mice in each group.

### DETAILED DESCRIPTION

The technical solutions of the present application are further described below via specific embodiments. It should be clear to those skilled in the art that the examples are merely used for a better understanding of the present application and should not be regarded as a specific limitation to the present application.

The Akk11 strain involved in the following examples has a taxonomic name of *Akkermansia muciniphila* Akk11 and a deposit number of CCTCC NO: M 2024119.

The BL21 strain involved in the following test examples has a taxonomic name of *Bifidobacterium longum* and a deposit number of CGMCC No.10452.

*Bifidobacterium longum* subsp. *Longum:* BNCC371780 strain.

The regular feed involved in the following examples is purchased from Shanghai Slac Laboratory Animal Co., Ltd; high-fat and high-sugar feed is purchased from Beijing Biotech HD Biotechnology Co., Ltd; Tris-HCl buffer is purchased from AMRESCO with product No. BS157-500 g; and skimmed milk is purchased from Fonterra Co-operative Group with the standard of fat less than or equal to 1.0% and protein more than or equal to 32.9%.

The MRS medium (g/L) involved in the following examples contains: peptone 10 g/L, beef paste 10 g/L, glucose 15 g/L, lactose 15 g/L, yeast powder 5 g/L, diammonium hydrogen citrate 2 g/L, K₂PO₄·3H₂O 2.6 g/L, MgSO₄·7H₂O 0.1 g/L, MnSO₄ 0.05 g/L, polysorbate 80 1 mL/L, cysteine monohydrochloride 0.5 g/L.

The bacterial suspension involved in the following examples is prepared by the following method: the Akk11 strain, BL21 strain and BNCC371780 strain are inoculated in skimmed milk and cultured at 37°C for 18 h for activation to obtain the activation liquid, respectively; the activation liquid is inoculated in the MRS medium at an inoculum amount of 4% (v/v) and cultured at 37°C for 20 h to obtain the bacterial liquid; the bacterial liquid is centrifuged at 8000 g for 10 min, and the supernatant is taken and filtered through a sterile filter membrane of 0.22 µm; and the bacterial cells are resuspended with PBS to give the bacterial suspension.

### Example 1

In this example, a strain of *Akkermansia muciniphila* with the function of regulating blood glucose and blood lipid is isolated and screened, and the steps are as follows.
(1) The fecal samples separated from healthy infants were diluted with 0.9% physiological saline for 10 times gradient, diluted for 3 times, coated on solid culture medium, cultured at 38°C for 48 h, picked out colonies with different forms, streaked and purified on the surface of improved MRS solid culture medium, picked out single colonies, expanded cultured with liquid culture medium at 37°C, and preserved with glycerol with 35% mass concentration.
(2) The physiological and biochemical characteristics of *Akkermansia muciniphila* in this example are characterized, and the results are shown in Table 1.

**Table 1**

| Test item | Result | Test item | Result |
|---|---|---|---|
| Gram staining | Negative | Cell shape | Rod-shaped |
| Catalase | + | Oxidase | - |

| Carbohydrates produce acid (API50CH) | | | |
|---|---|---|---|
| Glycerol | - | Esculin | - |
| Erythritol | - | Salicin | - |
| D-arabinose | - | Cellobiose | - |
| L-arabinose | - | Maltose | + |
| D-ribose | - | Lactose | + |
| D-xylose | - | Melibiose | - |
| L-xylose | - | Sucrose | - |
| Adonitol | - | Trehalose | - |
| β-methyl-D-xyloside | - | Inulin | - |
| D-galactose | + | Melezitose | - |
| D-glucose | + | Raffinose | - |
| D-fructose | + | Starch | - |
| D-mannose | + | Glycogen | - |
| L-sorbose | - | Xylitol | - |
| L-rhamnose | - | Gentiobiose | - |
| Melampyrite | - | D-turanose | - |
| Inositol | - | D-lyxose | - |
| Mannitol | - | D-tagatose | - |
| Sorbitol | - | D-fucose | - |
| α-methyl-D-mannoside | - | L-fucose | - |
| α-methyl-D-glucoside | - | D-arabite | - |
| N-acetyl-glucosamine | + | L-arabite | - |
| Amygdalin | - | Gluconate | - |
| Arbutin | - | 2-Keto-gluconate | - |

### Example 2

In this example, morphological identification and 16S rRNA molecular biological identification were carried out on the strains screened in Example 1, and the steps are as follows

### (1) Morphological identification

The strain was inoculated in MRS solid medium, cultured at 38°C for 48 h, and observed under a microscope. Microscopic examination after smear and Gram staining showed that Gram staining was negative, and the strain was non-motile, spore-free and rod-shaped.

### (2) Molecular biological identification of 16S rRNA

The strains preserved at -80°C were taken out, inoculated into a centrifuge tube filled with 20 ml of Mrs liquid culture medium at a ratio of 2%, cultured at 38°C for 18 h, then centrifuged for 10 min at 8000 rpm, and the supernatant was removed to collect the bacteria. The genome of the strain was extracted, and PCR amplification was carried out by adding universal bacterial primers, and the amplified products were sent to Sangon Biotech (Shanghai) Co., Ltd for sequencing and identification. The 16S rDNA sequence of this strain is shown in SEQ ID No:1 by sequencing analysis. The nucleic acid sequences of the sequenced sequences were compared in GeneBank, and the results showed that the strain was *Akkermansia muciniphila.*

SEQ ID No:1:

AGTCGAACGAGAGAATTGCTAGCTTGCTAATAATTCTCTAGTGGCGCACGGGTGAGT

AACACGTGAGTAACCTGCCCCCGAGAGCGGGATAGCCCTGGGAAACTGGGATTAAT

ACCGCATAGTATCGAAAGATTAAAGCAGCAATGCGCTTGGGGATGGGCTCGCGGCCT

ATTAGTTAGTTGGTGAGGTAACGGCTCACCAAGGCGATGACGGGTAGCCGGTCTGAG

AGGATGTCCGGCCACACTGGAACTGAGACACGGTCCAGACACCTACGGGTGGCAGC

AGTCGAGAATCATTCACAATGGGGGAAACCCTGATGGTGCGACGCCGCGTGGGGGA

ATGAAGGTCTTCGGATTGTAAACCCCTGTCATGTGGGAGCAAATTAAAAAGATAGTA

CCACAAGAGGAAGAGACGGCTAACTCTGTGCCAGCAGCCGCGGTAATACAGAGGTC

TCAAGCGTTGTTCGGAATCACTGGGCGTAAAGCGTGCGTAGGCTGTTTCGTAAGTCG

TGTGTGAAAGGCGCGGGCTCAACCCGCGGACGGCACATGATACTGCGAGACTAGAG

TAATGGAGGGGGAACCGGAATTCTCGGTGTAGCAGTGAAATGCGTAGATATCGAGAG

GAACACTCGTGGCGAAGGCGGGTTCCTGGACATTAACTGACGCTGAGGCACGAAGG

CCAGGGGAGCGAAAGGGATTAGATACCCCTGTAGTCCTGGCAGTAAACGGTGCACG

CTTGGTGTGCGGGGAATCGACCCCCTGCGTGCCGGAGCTAACGCGTTAAGCGTGCC

GCCTGGGGAGTACGGTCGCAAGATTAAAACTCAAAGAAATTGACGGGGACCCGCAC

AAGCGGTGGAGTATGTGGCTTAATTCGATGCAACGCGAAGAACCTTACCTGGGCTTG

ACATGTAATGAACAACATGTGAAAGCATGCGACTCTTCGGAGGCGTTACACAGGTGC

TGCATGGCCGTCGTCAGCTCGTGTCGTGAGATGTTTGGTTAAGTCCAGCAACGAGCG

CAACCCCTGTTGCCAGTTACCAGCACGTGAAGGTGGGGACTCTGGCGAGACTGCCC

AGATCAACTGGGAGGAAGGTGGGGACGACGTCAGGTCAGTATGGCCCTTATGCCCA

GGGCTGCACACGTACTACAATGCCCAGTACAGAGGGGGCCGAAGCCGCGAGGCGG

AGGAAATCCTAAAAACTGGGCCCAGTTCGGACTGTAGGCTGCAACCCGCCTACACG

AAGCCGGAATCGCTAGTAATGGCGCATCAGCTACGGCGCCGTGAATACGTTCCCGGG

TCTTGTACACACCGCCCGTCACATCATGGAAGCCGGTCGCACCCGAAGTATCTGAAG
CCAACCGCAAGGAGGCAGGGTCCTAAGGTGAGACTGGTAACTGGGATGAAGTCG.

Based on the results of molecular biological identification and morphological identification of 16S rRNA in Example 2, the strain was confirmed to belong to the *Akkermansia muciniphila,* and was named the *Akkermansia muciniphila* Akk11 strain.

### Test example 1

This test example tests the effect of Akk11 strain on slowing down weight gain, and the specific steps are as follows.
(1) Experimental animals: 42 6-week-old C57BL/6 male mice (25 g) were adaptively fed with regular feed for one week (free to diet and water) in an animal house (room temperature: 25°C, humidity: 50%, lighting for 10 h).
(2) Grouping: after adaptive feeding, mice were randomly assigned to 7 groups: normal-control group, normal-experimental group, high-fat-Akk11 group, high-fat-BL21 group, high-fat-Akk11 + BL21 combined group, high-fat-Akk11 + BNCC371780 combined group, and high-fat-control group, and 6 mice in one group.

### (3) Intervention method

### (3.1) Regular feed group

Control group: fed with regular feed for 30 days and gavaged with 0.5 mL of PBS buffer once a day for 30 days.

Experimental group: fed with regular feed for 30 days and gavaged with 0.5 mL of the bacterial suspension of Akk11 (concentration of 2 × 10⁹ CFU/mL) once a day for 30 days.

### (3.2) High-fat and high-sugar feed group

Mice were fed with high-fat and high-sugar feed, and gavaged with any one group of the following liquid once a day respectively, for 30 days to form different experimental groups: 1) bacterial suspension of Akk11 for 0.5 mL (concentration of 2 × 10⁹ CFU/mL); 2) bacterial suspension of BL21 for 0.5 mL (concentration of 2 × 10⁹ CFU/mL); 3) combined bacterial suspension of Akk11 and BL21 for 0.5 mL (total concentration of 2 × 10⁹ CFU/mL, a viable bacteria count ratio of 1:1); 4) combined bacterial suspension of Akk11 and BNCC371780 for 0.5 mL (total concentration of 2 × 10⁹ CFU/mL, a viable bacteria count ratio of 1:1 ); and 5) control group: PBS buffer for 0.5 mL.

Mice in all groups were fed freely with feed and drinking water,, and the feeding environment was an animal house (room temperature: 25°C, humidity: 50%, lighting for 10 h) for 30 consecutive days, and at the end of the 30th day, the weight of each mouse in each group was weighed, the average body weight growth rate of each group was calculated respectively, and the regulating effect of the bacterial preparation on the obesity of mice was evaluated on the basis of the body weight growth rate. Body weight growth rate (%) = (the weight of mice in each group at the end of the experiment of the 30th day - initial weight before the experiment) / initial weight before the experiment × 100%, and the average body weight growth rate (%) = Sum of weight growth rates of mice in each group / number of mice per group. The results are shown in Table 2.

**Table 2**

| Group No. | Average body weight growth rate (%) |
|---|---|
| Normal-control group | 46.52 |
| Normal-experimental group (Akk11) | 46.31 |
| High-fat-Akk11 group | 50.16 |
| High-fat-BL21 group | 55.34 |
| High-fat-Akk11 + BL21 combined group | 46.84 |
| High-fat-Akk11 + BNCC371780 combined group | 52.35 |
| High-fat-control group | 126.25 |

From Table 2, it can be seen that both Akk11 and BL21 bacterial suspensions can slow down the weight growth rate of obese mice, and Akk11 bacterial suspension has the best slowing effect, and the weight growth rate of mice shows an obvious decreasing trend, which is close to that of normal-control mice. In addition, it was unexpectedly found that when Akk11 and BL21 were used together, the effect of slowing down the weight growth rate of obese mice was better, and the weight growth rate of mice was lower.

### Test example 2

This test example tests the ability of the Akk11 strain to decompose and utilize glucose, and the detailed steps were as follows

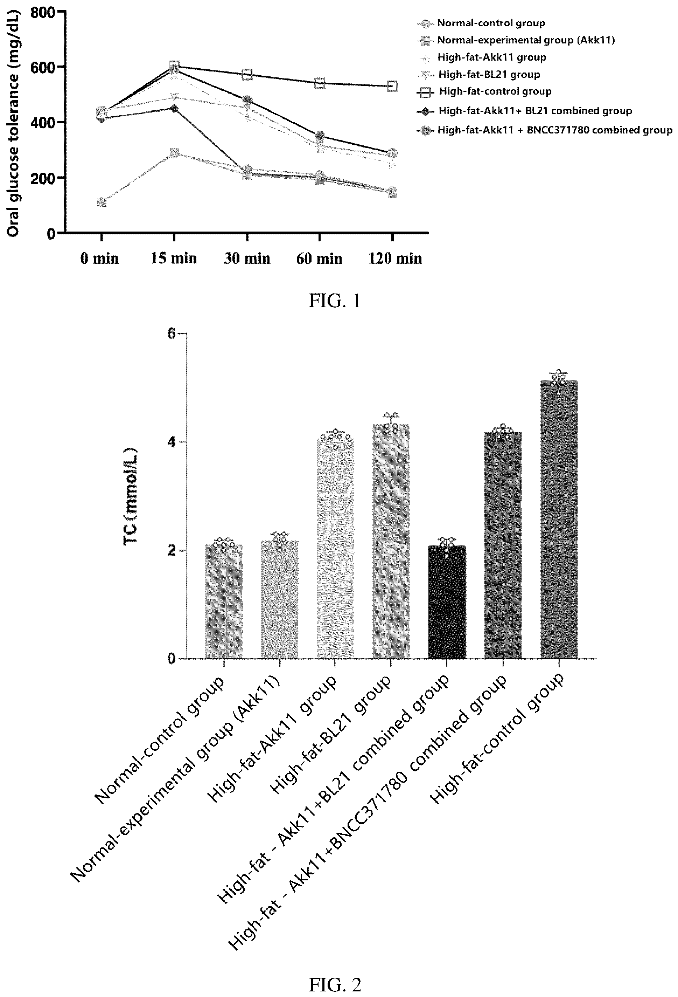

Grouping and experimental operations are the same as those in Test Example 1. After 30th day of the experiment, oral glucose tolerance (OGTT) was measured after the mice were fasted for 6 h. The mice were fed 1.5 mg/kg glucose orally according to their body weights, and blood was extracted from the tails of the mice at 0 min, 15 min, 30 min, 60 min, and 120 min, blood glucose was measured by using a glucometer, and the curves of the changes of oral glucose tolerance over time were plotted as shown in FIG. 1.

The results showed that glucose tolerance of the high-fat-control group is higher than those of the normal-control group and the normal-experimental group, which proved that mice fed with high-fat and high sugar feed have a disturbed regulation of glucose metabolism levels in their bodies. After injecting 15 min/kg glucose, the serum glucose level increases sharply, and then decreases. Although the oral glucose level decreases during 15-120 min, the decrease rate is slow, which indicates that the mice fed with high-fat and high-sugar feed have weak ability to decompose and utilize glucose. Both Akk11 and BL21 bacterial suspensions can improve the conditions of glucose metabolism disorders in obese mice, and the values of glucose tolerance of mice in all time periods are significantly decreased, and the oral glucose level is obviously decreased during 15-120 min, among which Akk11 bacterial suspension has the best improvement effect. In addition, the combination of Akk11 and BL21 can further improve the ability of glucose decomposition and utilization in mice, and the ability to improve the disorder of glucose metabolism in obese mice is the best.

### Test example 3

This test example tests the ability of the Akk11 strain to regulate blood lipid, and the detailed steps are as follows.

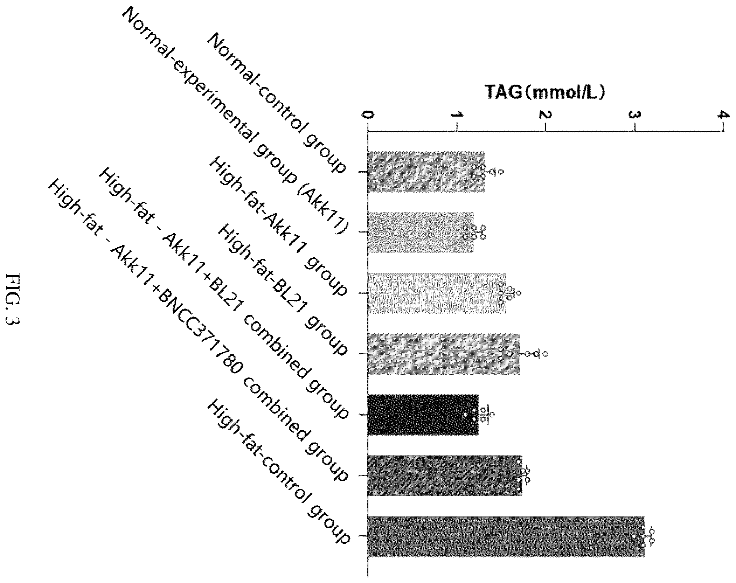

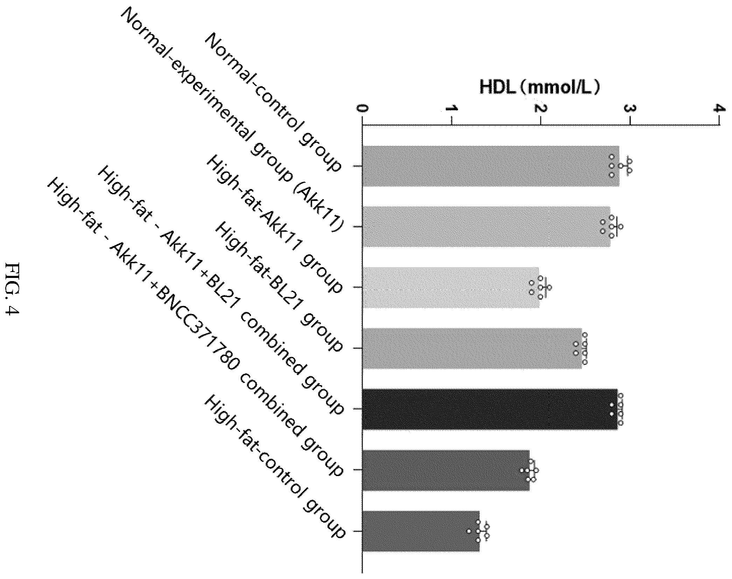

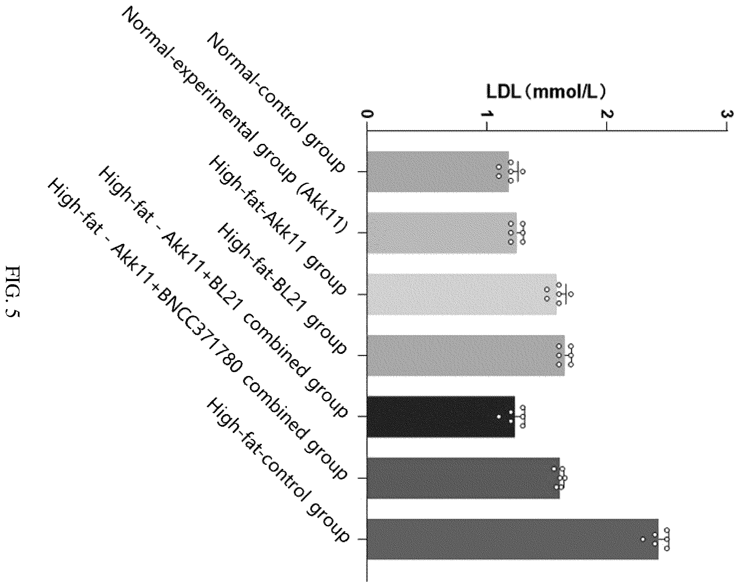

Grouping and experimental operations are the same as those in Test Example 1. After the 30th day of the experiment, vein blood was taken from the tail of mice, centrifuged at 2000 rpm for 10 min and the serum was taken. The contents of total cholesterol (TC), triglyceride (TAG), high-density lipoprotein (HDL), and low-density lipoprotein (LDL) in the serum of mice in each group were detected by using a fully automatic biochemical analyzer, and the results are shown in FIGS. 2-5.

As can be seen from FIGS. 2-5, both Akk11 and BL21 bacterial suspensions can reduce total cholesterol, triglycerides and low-density lipoprotein of mice, all of which are lower than those of the high-fat control group, while the high-density lipoprotein is higher than that of the high-fat control group. The effect of Akk11 bacterial suspension is better than that of BL21 bacterial suspension, and all indexes are close to those of the normal control group. In addition, it is unexpectedly found that when Akk11 and BL21 are used in combination, it is better to reduce the indexes such as total cholesterol, triglyceride and low-density lipoprotein cholesterol, so as to improve the symptoms of blood lipid metabolism disorder in mice.

### Test example 4

This test example tests the ability of the Akk11 strain to improve the inflammatory response, and the detailed steps are as follows.

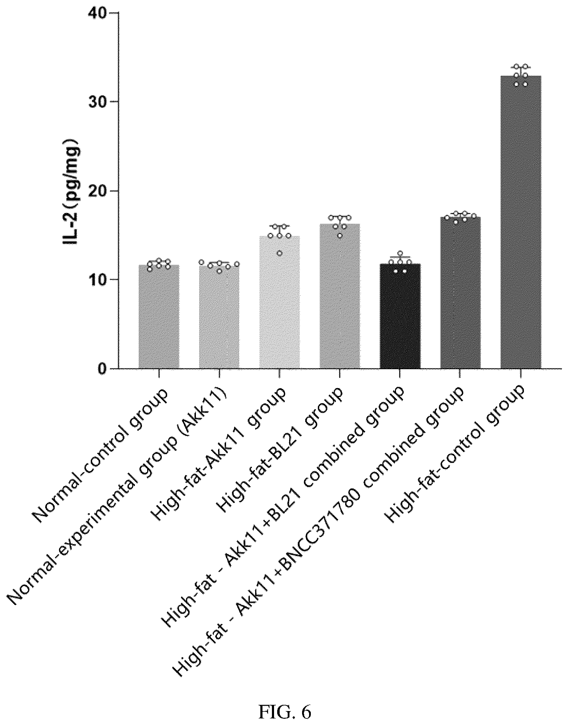

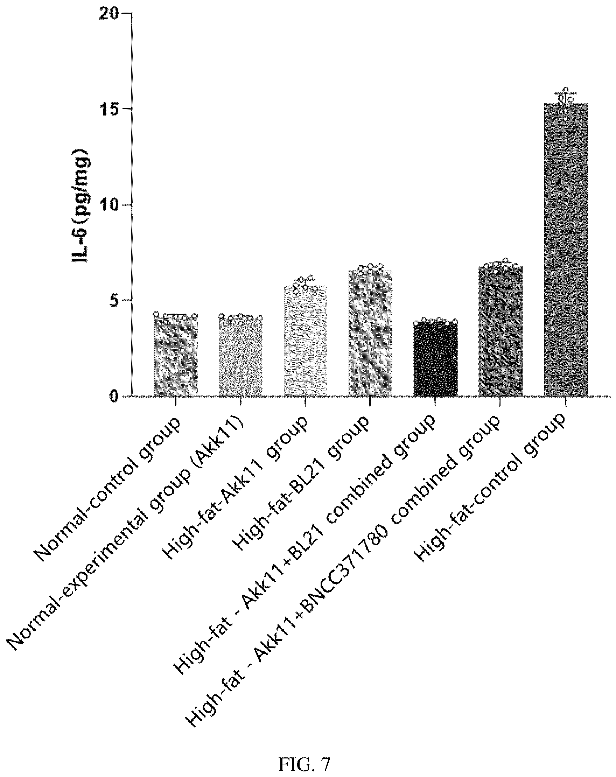

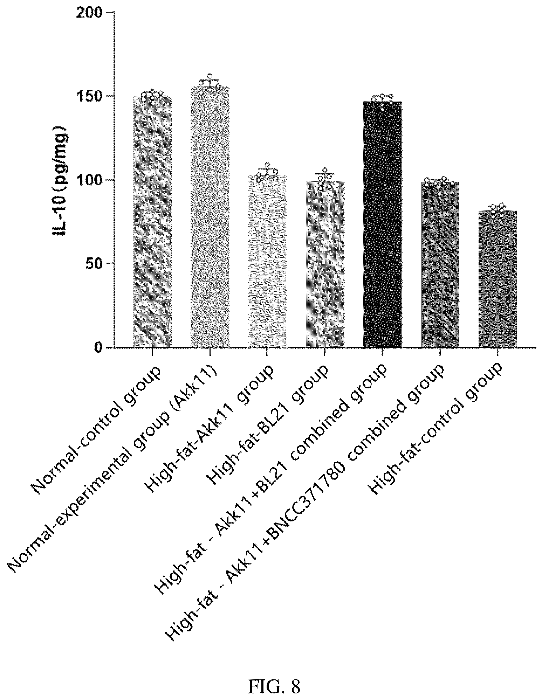

Grouping and experimental operations are the same as those in Test Example 1 After the 30th day of the experiment, mice were dissected, and pancreatic tissue was removed and homogenized using Tris-HCl buffer (pH = 7.4). The homogenates were centrifuged at 1800 g at 4°C for 10 min, and the supernatant was collected and analyzed for protein concentration and cytokines. Protein concentration was measured by Coomassie blue staining; and cytokine interleukin-2 (IL-2), interleukin-6 (IL-6), and interleukin-10 (IL-10) levels were determined by ELISA (mouse cytokine kit, Wuhan Chundu Biotech Co., Ltd), and the results are shown in FIGS. 6-8.

As can be seen from FIGS. 6-8, the values of interleukin-2 and interleukin-6 in the mice of the high-fat control group fed with high-sugar and high-fat feed are significantly higher than those in the normal-control group fed with regular feed, and the level of interleukin-10 was significantly lower than that of the normal-control group, indicating that the gastrointestinal -inflammation of obese mice fed with high-fat and high-sugar feed is more severe than that in the normal control group.

The Akk11 and BL21 bacterial suspensions both can reduce the levels of interleukin-2 and interleukin-6 and increase the level of interleukin-10, indicating that the probiotic suspension can effectively regulate the gastrointestinal inflammation of obese mice and has a significant improvement effect on inflammatory factors, and the adjustment effect of Akk11 bacterial suspension is the best. In addition, when Akk11 and BL21 strains are used in combination, the effect of improving the levels of interleukin-2, interleukin-6 and interleukin-10 is better.

To sum up, in the present application, a new strain of *Akkermansia muciniphila* with the effect of regulating blood glucose and blood lipid is isolated and deposited, which is named as *Akkermansia muciniphila* Akk11. The strain has significantly improved the excessive weight growth rate, blood lipid metabolism disorder, and gastrointestinal inflammation in obese mice, indicating that the *Akkermansia muciniphila* Akk11 described in the present application is very effective in regulating metabolism-related symptoms in mice. Therefore, the *Akkermansia muciniphila* Akk11 strain can be used in the preparation of a product with the effect of improving obesity.

The applicant declares that the present application illustrates the technical solutions of the present application by the above embodiments, but the present application is not limited to the above embodiments, that is, the present application does not necessarily rely on the above embodiments to be implemented. Those skilled in the art should understand that any improvements of the present application, the equivalent substitution of each raw material, the addition of auxiliary ingredients, and the selection of specific methods shall fall within the protection scope and disclosure scope of the present application.

The above describes in detail the preferred embodiments of the present application. However, the present application is not limited to the specific details in the above embodiments, and various simple variations of the technical solutions of the present application can be made within the scope of the technical conception of the present application, all of these simple variations shall fall within the protection scope of the present application.

It is also to be noted that the various specific technical features described in the above specific embodiments may be combined in any suitable manners without contradiction, and in order to avoid unnecessary repetition, the various possible combinations are not described separately in the present application.

## Claims

1. An *Akkermansia muciniphila* Akk11 with the effect of regulating blood glucose and blood lipid, which is taxonomy named as *Akkermansia muciniphila* Akk11 and deposited on Jan. 15, 2024, with a deposit number CCTCC NO: M 2024119.

2. A culture of the *Akkermansia muciniphila* Akk11 with the effect of regulating blood glucose and blood lipid according to claim 1, which is prepared by the following method: inoculating the *Akkermansia muciniphila* Akk11 strain in a medium and culturing at 35-38°C for 18-22 h.

3. A probiotic preparation with the effect of regulating blood glucose and blood lipid, which comprises the *Akkermansia muciniphila* Akk11 strain according to claim 1.

4. The probiotic preparation with the effect of regulating blood glucose and blood lipid according to claim 3, wherein a viable bacteria count of the *Akkermansia muciniphila* Akk11 strain in the probiotic preparation is not less than 1×10¹⁰ CFU/mL or 1×10¹⁰ CFU/g.

5. The probiotic preparation with the effect of regulating blood glucose and blood lipid according to claim 3, wherein the probiotic preparation with the effect of regulating blood glucose and blood lipid further comprises *Bifidobacterium longum* BL21, and the *Bifidobacterium longum* BL21 is taxonomy named as *Bifidobacterium longum* and deposited on Jan. 27, 2015, with a deposit number CGMCC No.10452.

6. The probiotic preparation with the effect of regulating blood glucose and blood lipid according to claim 5, wherein a viable bacteria count ratio of the *Akkermansia muciniphila* Akk11 strain to the *Bifidobacterium longum* BL21 strain is (1:10)-(10:1).

7. The probiotic preparation with the effect of regulating blood glucose and blood lipid according to claim 3, wherein a dosage form of the probiotic preparation comprises a lyophilized powder agent, a capsule agent, a tablet agent or a granule agent.

8. The probiotic preparation with the effect of regulating blood glucose and blood lipid according to claim 3, wherein the probiotic preparation further comprises a protectant and/or a functional additive;
the protectant comprises any one or a combination of at least two of skimmed milk, gelatin, dextrine, Arabic gum, dextran, sodium algae, polyvinyl pyrrolidone, sucrose, lactose, trehalose, sorbitol or xylitol; and
the functional additive comprises any one or a combination of at least two of maltodextrin, oligofructose, oligogalactose, oligoxylose, soybean oligosaccharide, inulin, *Spirulina, Arthrospira,* Coriolus versicolor polysaccharide, stachyose, polyglucose, α-lactalbumin, or lactoferrin.

9. Use of the *Akkermansia muciniphila* Akk11 with the effect of regulating blood glucose and blood lipid according to claim 1, or the culture according to claim 2, or the probiotic preparation according to any one of the claims 3-8 in the preparation of a product with the effect of preventing, improving or treating obesity, hyperlipidemia, and hyperglycemia.

10. The use according to claim 9, wherein the product further comprises an auxiliary material; the auxiliary material comprises any one or a combination of at least two of a diluent, an excipient, a binder, a wetting agent, a disintegrant, an emulsifier, a cosolvent, a solubilizer, an osmotic pressure regulator, a coating material, a colorant, a pH regulator, or a buffer.
